(19) Europäisches Patentamt
European Patent Office
Office européen des brevets

(11) **EP 3 050 430 A1**

(12) **EUROPEAN PATENT APPLICATION**

(43) Date of publication:
**03.08.2016 Bulletin 2016/31**

(21) Application number: **16151852.7**

(22) Date of filing: **19.01.2016**

(51) Int Cl.:
*A01J 5/01* (2006.01)     *B67D 7/08* (2010.01)
*G01F 1/74* (2006.01)     *G01F 15/08* (2006.01)
*G01F 23/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **26.01.2015 DK 201500040**

(71) Applicant: **VM Tarm A/S**
**6880 Tarn (DK)**

(72) Inventors:
• **Berg-Konstmann, Kristian**
  **6800 Varde (DK)**
• **Skovbjerg, Jan**
  **6818 Årre (DK)**
• **Thomsen, Marius**
  **6900 Skjern (DK)**

(74) Representative: **Nielsen, Charlotte**
**Tropa ApS**
**Aagade 97**
**8370 Hadsten (DK)**

(54) **DEVICE AND METHOD FOR COLLECTING A FLUID**

(57)     A method for measuring the quantity of fluid received by the detection device is disclosed. The method comprises the following steps:
- connecting the detection device (2) to an inlet tank (4);
- filling a quantity of fluid (46) from the inlet tank (4) into the detection device (2), wherein the quantity of fluid (46) exceeds a predefined volume;
- closing an outer valve (12) and flooding a portion of the detection device (2) hereby evacuating/removing the air in this portion of the detection device (2);
- detecting the fluid level ($L_1$) in the flooded portion of the detection device (2).

The method comprises the step of filling fluid (46) into a gas separator (24) and detecting the fluid level in the gas separator (24).

The method comprises the step of:
- opening the outer valve (12) and filling a further quantity of fluid (46) from the inlet tank (4) into the detection device (2), while detecting the flow and/or volume of the fluid (46);
- emptying the inlet tank (4);
- closing an outer valve (12) and flooding a portion of the detection device (2) hereby evacuating/removing the air in this portion of the detection device (2) and
- detecting the fluid level in the flooded portion of the detection device (2).

The method comprises the step of applying one or more pumps (18, 20) to transport fluid (46) into and/or within the detection device (2).

The method comprises the step of determining the pressure at both sides of a pressure difference inducing member (28) provided in the detection device (2), wherein the method further comprises the step of determining the flow (Q1, Q2) at each side of the pressure difference inducing member (28), and applying the pressures and flows at both sides of the pressure difference inducing member (28) to calculate the gas content of the fluid (46).

Fig. 1

## Description

### Field of invention

The present invention generally relates to a method and use of said for collecting a fluid. The present invention more particularly relates to a method for collecting a fluid from a tank and transporting it to a tanker or collecting a fluid from a tank and transporting it to another tank. The present invention further relates to a detection device which may be used or applied in the method of the invention.

### Prior art

In WO 83/04407 A1, a procedure for gathering milk is described. The procedure comprises measuring the volume of the milk by separating milk and air using pump means and an air separator. Thus, the volume of milk may be measured with less errors for larger quantities, however, such procedure may not be suited for smaller quantities.

In GB 2 432 680 A, an apparatus and a method for conveying and metering a liquid such as milk are disclosed, by which air is removed from the mixture using a eliminator vessel system. Thus, it is an object to provide an improved system to handle the milk more quickly, however, such may not be suited for smaller quantities.

In GB 2 240 764 A, an apparatus and a method for batch metering of milk transferred between two containers are described. The system makes use of an air detector probe to determine the amount of milk and air, respectively, as well as gas venting means. However, the procedure in connection with the gathering of milk is time-consuming and involves many routines, thus, making the system not suited for smaller quantities or collection of milk at various sites.

In EP 808 51 A1, an air separator for milk tankers is disclosed. Said air separator comprises a regulating device for continuous operation of the pump effect. However, this method is not very suited when determining the amount of milk in a milk/air mixture.

During the process of filling a tanker with a fluid from a tank, it is of great importance to keep track of how much fluid the system including the inlet hose, the pipes, and the gas separator of the detection device and/or tanker contains. Taking these volumes of fluid into consideration may be crucial for the purpose of providing an accurate measurement of the collected fluid, especial when handling small amounts of fluid (e.g. below 300 litres).

The prior art systems for collecting fluids such as milk from farmers are not capable of taking all these volumes into consideration. Therefore, the current tankers available on the market are not capable of providing liquid measurement with sufficient accuracy.

Accordingly, it would be desirable to have a device and a method capable of providing an improved and more accurate measurement of the collected fluid.

Thus, it is an object of the present invention to provide a method and a detection device capable of providing accurate, fluid collection detection as well as use of said method in a tanker.

It is also an object of the present invention to provide a method for collection of small quantities of fluid, which allow high accuracy measurements of the fluid volume. It is also an object of the present invention to provide a tanker configured to carry out the method according to the invention.

### Summary of the invention

The object of the present invention can be achieved by a method as defined in claim 1, by a use as defined in claim 9, a tanker as defined in claim 11, and a detection device as defined in claim 15. Preferred embodiments are defined in the dependent sub claims, explained in the following description and illustrated in the accompanying drawings.

The method according to the invention is a method for measuring the quantity of fluid received by a detection device, which method comprises the following steps:

- connecting the detection device to an inlet tank;
- filling a quantity of fluid from the inlet tank into the detection device, wherein the quantity of fluid exceeds a predefined volume;
- closing an outer valve and flooding a portion of the detection device hereby evacuating/removing the air in this portion of the detection device;
- detecting the fluid level in the flooded portion of the detection device.

Hereby, it is possible to provide accurate, fluid collection detection even if small quantities of fluid are collected.

The method is capable of measuring the quantity of fluid received by the detection device of e.g. a tanker or another device. The method may be applied to measure fluids such as milk, oil, gasoline, water or other fluids or a combination of one or more thereof. The method may be applied for the detection of fluids comprising a mixture of liquid and gas. The term "mixture of liquid or gas" thus applies to such containing one or more liquids as well as one or more gasses. The mixture of liquid and gas may further contain solid constituents optionally dispersed in the liquid(s). Examples of such solid constituents are fatty components in milk. Examples of gasses are air.

The method comprises the step of connecting the detection device to an inlet tank. This may be done by mechanically connecting an inlet hose or an inlet pipe to an inlet tank by any suitable means.

By flooding a portion of the detection device, it is possible to evacuate and hereby remove the air in this portion of the detection device.

Accordingly, it is possible to detect the volume of the fluid in the flooded portion of the detection device. The prior art devices cannot make a detection of the same accuracy as a significant volume within the prior art detection devices is filled with mixture of liquid and gas during the initial detection phase. Thus, one cannot determine the liquid or gas percentages of this fluid in the prior art detection devices.

The method comprises the step of connecting the detection device to an inlet tank. This may be done in any suitable manner by means of any appropriate connection means. Typically, a hose is connected to the inlet tank.

The method comprises the step of filling a quantity of fluid from the inlet tank into the detection device, wherein the quantity of fluid exceeds a predefined volume. The fluid volume required depends on the geometry and size of the detection device.

By closing an outer valve and flooding a portion of the detection device and hereby evacuating/removing the air in this portion of the detection device, it is possible to determine the quantity of fluid in the detection device during this initial phase. The determination of the quantity of fluid in the detection device is carried out by detecting the fluid level in the flooded portion of the detection device. This may be done by means of any suitable detection members including level sensors.

During the flooding process, the fluid will flow backwards and fill the lowest portion of the detection device. The fluid cannot escape as the outer valve is closed.

The method further comprises the step of filling fluid into a gas separator and detecting the fluid level in the gas separator.

The use of a gas separator is an advantage. Detecting the fluid level in the gas separator is an easy way of detecting the fluid level in the flooded portion of the detection device. Moreover, the gas content of the fluid can be evacuated/removed.

Flooding the detection device by filling a quantity (within a predefined range) of fluid from the inlet tank into the detection device and evacuating the air from the fluid, makes it possible to measure and take this volume into consideration during the further process steps. Hereby, the accuracy of the method can be improved compared to the prior art methods.

Flooding may be conducted by filling fluid from the inlet tank into a central or lower portion of the detection device.

It may be an advantage that the method comprises the step of:

- opening the outer valve and filling a further quantity of fluid from the inlet tank into the detection device, while detecting the flow and/or volume of the fluid;
- emptying the inlet tank;
- closing an outer valve and flooding a portion of the detection device hereby evacuating/removing the air in this portion of the detection device and
- detecting the fluid level in the flooded portion of the detection device.

In some embodiments, the method may comprise a first step, wherein the mixture to be determined is passed through a gas separator.

Hereby, it is possible to compare the detected fluid levels and calculate the total volume of fluid detected by the detection device in an accurate manner.

It may be an advantage that the method comprises the step of determining the pressure at both sides of a pressure difference inducing member provided in the detection device, wherein the method further comprises the step of determining the flow at each side of the pressure difference inducing member and applying the pressures and flows at both sides of a pressure difference inducing member to calculate the air/gas content of the fluid.

Hereby, an even more accurate determination of the volume of the fluid (and its liquid portion) can be achieved.

The method may further comprise the step of detecting one or more parameters defining the quantity of fluid filled into the detection device. Such parameters may be e.g. the fluid level in a gas separator or other suitable parameters.

The method may further comprise the step of filling a further quantity of fluid from the inlet tank into the detection device, while detecting the flow and/or volume of the fluid. This process step may be carried out by any suitable means including valves, pumps and sensors including flow sensors.

It may be preferred that the method comprises the step of emptying the inlet tank completely and detecting the remaining quantity of fluid in the detection device.

It may be an advantage that the method comprises the step of filling fluid into a gas separator (within the detection device) and detecting the fluid level in the gas separator.

By filling fluid into a gas separator and detecting the fluid level of the gas separator, the quantity of fluid in the gas separator can be determined in such a manner that this quantity can be taken into consideration during the further steps of the filling process.

When fluid is filled into the gas separator, fluid will also be filled into one or more pipe structures and/or further components (pumps, valves or sensors). As the volume of these components is known, it is possible to detect the exact quantity of fluid stored in the detection device. Accordingly, an accurate measurement of the collected quantity of fluid can be achieved.

It may be advantageous that the method comprises the step of flooding a portion of the detection device when an inlet tank has been emptied, and the remaining quantity of fluid in the detection device has been detected (measured).

By flooding a portion of the detection device when an inlet tank has been emptied, and the remaining quantity of fluid in the detection device has been detected, it is possible to achieve a "known and well-defined" start condition when collecting fluid from the "next" inlet tank (e.g. the next farmer delivering milk).

It may be an advantage to flood a predefined portion of the detection device, e.g. a portion of the gas separator and a selection of the pipe structures supplying fluid to and from the gas separator.

It may be beneficial that the method comprises the step of emptying an inlet hose (or inlet pipe) connected to the inlet tank.

By emptying an inlet hose (or inlet pipe) connected to the inlet tank, it is possible to eliminate the error introduced by not knowing the volume of fluid stored within the inlet hose.

It may be advantageous that the method comprises the step of applying one or more pumps to transport fluid into and/or within the detection device.

By applying one or more pumps to transport fluid into and/or within the detection device, it is possible to provide fast fluid transport. Moreover, the one or more pumps may be applied to control the access of the fluid to one or more structures in the detection device (when a pump is deactivated, it may function as a valve).

It may be beneficial that the method comprises the step of emptying or filling the one or more pumps.

By emptying or filling the one or more pumps, it is possible to provide a known and well-defined condition, which makes it easier to conduct an additional fluid collection and still provide high accuracy.

It may be an advantage that the method comprises the step of detecting pressure at both sides of a pressure difference inducing member provided in the detection device, wherein the method further comprises the step of determining the flow at each side of the pressure difference inducing member and applying the pressures and flows at both sides of a pressure difference inducing member to calculate the air/gas content of the fluid.

Hereby, it is possible to provide an accurate detection of the liquid portion of the collected fluid.

It may be an advantage that the method comprises the step of detecting a first flow at a first side of the pressure difference inducing member by means of a first flow sensor arranged at the first side of the pressure difference inducing member.

It may be beneficial that the method comprises the step of detecting a second flow at the other side of the pressure difference inducing member by means of a second flow sensor arranged at the other side of the pressure difference inducing member.

It may be an advantage that the method comprises the step of determining the gas content of the liquid-containing fluid by means of the flow measurements made by the flow sensors together with pressure information (e.g. detected by means of pressure sensors).

It may be advantageous that the method comprises the step of measuring the pressure difference across the pressure difference inducing member by means of one or more pressure sensors, e.g. by means of a single pressure difference sensor.

It may be an advantage that the pressure difference inducing member is a valve or a pump or a pipe or a motor.

It may be beneficial that the measurements are continuous measurements or intermittent measurements.

Hereby, it is possible to carry out accurate measurements of a fluid which is received by the detection device.

In some embodiments, a pump is not needed. The filling member may merely consist of the pipe structures in case the inlet tank is pressurised.

The outer valve is configured to prevent fluid from flowing back into the inlet tank during the flooding process. Hereby, the air inside the flooded portion of the detection device will be evacuated/removed.

The flow sensors are flooded during the flooding process, which enables the flow sensors to provide flow measurements.

The detection device according to the invention may preferably be used in a tanker for measuring the quantity of fluid received by the detection device, and the device comprises:

- a gas separator and a level detection member configured to detect the fluid level ($L_1$, $L_2$, $L_3$) of the gas separator;
- a connection member for connecting the detection device to an inlet tank;

- a filling member for filling a quantity (within a predefined range) of fluid from the inlet tank into the detection device, the quantity of fluid exceeding a predefined volume;

- an outer valve arranged between the filling member and the distal end of the connection member;
- a first flow sensor and a second flow sensor arranged below the gas separator;
- a main pump adapted to pump fluid from the inlet tank into and through the detection device;
- a feeder pump connected to the main pump via a pipe;
- a valve arranged between the main pump and the gas separator, which valve is adapted to control the degree of access to a bypass pipe bypassing the feeder pump via the pipe;
- a valve provided in order to close and open an outlet pipe of the gas separator for the purpose of flooding the pump.

The detection device applied and used in accordance with the present invention may further comprise:

- one or more detection members for detecting the remaining quantity of fluid in the detection device when the inlet tank has been emptied;
- a detection member for detecting one or more parameters defining the quantity of fluid filled into the detection device.

Hereby, it is possible to provide accurate fluid collection detection even when a small quantity of fluid is collected.
It may be an advantage that the detection device used in the invention comprises means for flooding a portion of the detection device, when an inlet tank has been (at least partly) emptied, and the remaining quantity of fluid in the detection device has been detected. Flooding the "system" will provide a "well-defined" and "known" start condition for the following measurement.
Accordingly, the detection device is filled in a "well-defined" manner, in which the quantity of fluid stored in the detection device can be taken into consideration so that the next quantity of fluid being collected can be measured with high accuracy.
The object of the invention can be achieved by the method according to one of the claims 1-8 or the use according to claim 9-10.
The tanker according to the invention is a tanker comprising a detection device for measuring the quantity of fluid received by the detection device, wherein the detection device is configured carry out the method according to the invention, wherein the detection device is configured to be connected to an inlet tank, wherein the detection device is configured to receiving a quantity of fluid from the inlet tank being filled into the detection device, wherein the quantity of fluid exceeds a predefined volume, wherein the detection device comprises an outer valve configured to be brought from an open configuration into a closed configuration, wherein the detection device is configured to be at flooded in at least a portion of the detection device in order to evacuate/remove the air in this portion of the detection device, wherein the detection device comprises means for detecting the fluid level in the flooded portion of the detection device.
Hereby, it is possible to provide a tanker capable of carrying out an accurate, fluid collection even if small quantities of fluid are collected.
It may be beneficial that the detection device comprises a gas separator and means for detecting the fluid level in the gas separator, wherein the gas separator is configured to receive fluid being filled into the gas separator. Hereby, the tanker is capable of evacuating air/gas in the gas separator and at the same time taking into account the fluid level in the gas separator in or der to provide high precision measurements of the quantity of fluid received by the detection device.
It may be an advantage that the tanker comprises means for detecting the flow and/or volume of a fluid filled from the inlet tank into the detection device. Hereby, valid measurements can be established.
It may be advantageous that the tanker comprises one or more pumps arranged and configured to transport fluid into and/or within the detection device. Hereby, the circulation of fluid may be provided within desired flow ranges in order to meet predefined flow ranges.

Description of the Drawings

The invention will become more fully understood from the detailed description given herein below. The accompanying drawings are given by way of illustration only, and thus, they are not limitative of the present invention. In the accompanying drawings:

Fig. 1      shows a schematic view of a detection device integrated within a tanker;
Fig. 2 A)      shows a detection device corresponding to the one shown in Fig. 1;
Fig. 2 B)      shows the detection device shown in Fig. 2A in a configuration in which fluid has entered the inlet hose, the gas separator and a number of pipes;

Fig. 2 C)  shows the detection device shown in Fig. 2 A) and Fig. 2 B) in a configuration in which the fluid level is registered by the control unit;

Fig. 2 D)  shows the detection device in a configuration in which the detection device is transported to the next farmer to collect milk from a new inlet tank;

Fig. 3 A)  shows a schematic view of a detection device, in which the valve 28 is arranged in its "floating position";

Fig. 3 B)  shows a schematic view of the detection device shown in Fig. 3 A), in which the feeder pump 20 is activated, and the valve 12' is closed.

Fig. 3 C)  shows a schematic view of the detection device shown in Fig. 3 A), in which the valves 12, 28, 12" are closed, whereas the valves 12', 16 are open;

Fig. 3 D)  shows the final process step, in which the valves are opened in order to flood the central portion of the detection device and

Fig. 4  shows a schematic view of a detection device integrated within a tanker.

## Detailed description of the invention

Referring now in detail to the drawings for the purpose of illustrating preferred embodiments of the present invention.

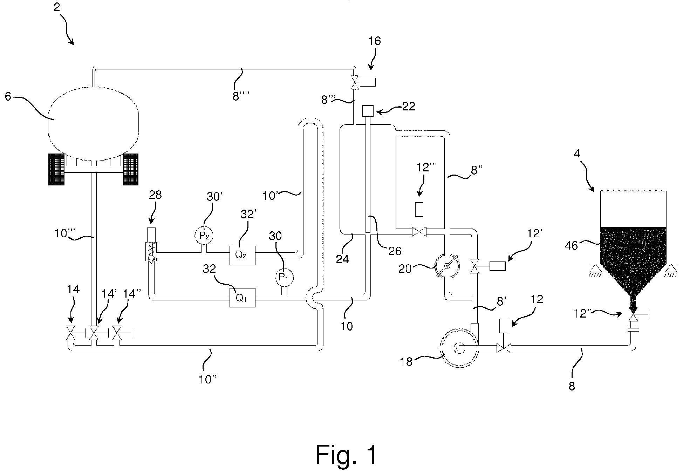

Fig. 1 illustrates a schematic view of a detection device 2 integrated within a tanker. The tanker comprises a container 6 configured to be brought into fluid communication with an (external) inlet tank 4. The inlet tank 4 may be e. g. a milk tank applied for milk storage at a farm.

The inlet tank 4 comprises a pipe 8 and a control valve 12" configured to be fully or partially opened or closed in order to adjust the fluid flow.

At the end of the pipe 8, a control valve 12 is provided. The pipe 8 is connected to a main pump 18 adapted to pump fluid 46 from the inlet tank 4 into and through the detection device 2.

The detection device 2 comprises a gas separator 24 which is shaped as a tank provided with a dipstick 22 having a stick member 26. A pipe 8' connects the main pump 18 to a feeder pump 20 and a valve 12' controlling the degree of access to a bypass pipe (bypassing the feeder pump 20). An outlet pipe is provided at the bottom portion of the gas separator 24, and a valve 12'" is provided in order to close and open the outlet pipe (for the purpose of flooding the pumps 18, 20). A pipe 8" is connected to the upper portion of the gas separator 24.

An inlet pipe 10 connects the bottom portion of the gas separator 24 with a first pressure sensor 30 of the detection device 2. A first flow sensor 32 is provided next to the first pressure sensor 30. The first pressure sensor 30 detects a first pressure $P_1$, whereas the first flow sensor 32 detects a first flow $Q_1$.

A second pressure sensor 30' configured to detect a second pressure $P_2$ is arranged in fluid communication with the first flow sensor 32, and a check valve 28 is provided between the first flow sensor 32 and the second pressure sensor 30'. A second flow sensor 32' adapted to measure a second flow $Q_2$ is arranged next to the second pressure sensor 30'. The sensor configuration corresponds to the one illustrated in Fig. 1A.

The second flow sensor 32' is provided at the outlet pipe 10' which is in fluid communication with a connection pipe 10" provided with three end valves 14, 14', 14" each configured to be connected to an inlet pipe 10'" which is connected to a container 6 of the tanker.

An air evacuation pipe 8"" is provided at the upper portion of the container 6 of the tanker. The evacuation pipe 8"" is connected to the gas separator 24 through a connection pipe 8'". A valve 16 connects the evacuation pipe 8"" and the connection pipe 8'".

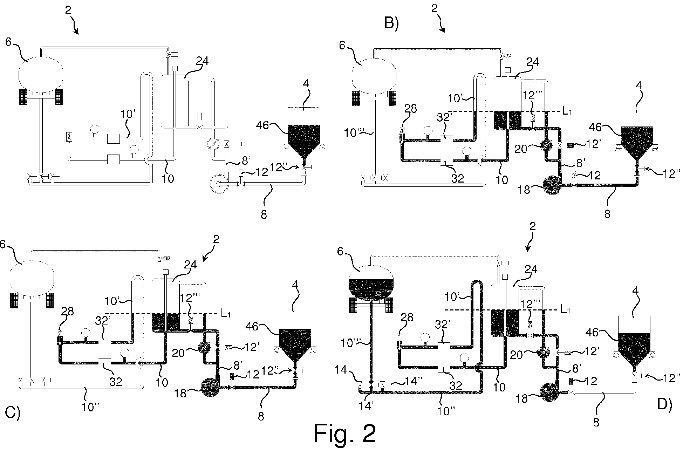

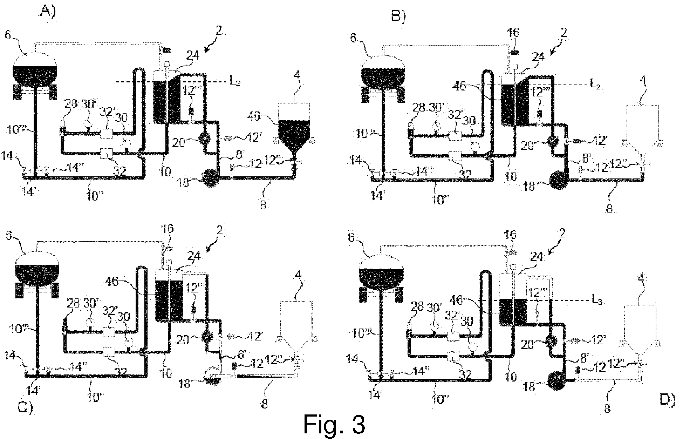

As it will be explained in further detail with reference to the following figures (Fig. 2 and Fig. 3), the detection device 2 according to the invention makes it possible to detect the fluid 46 and the air content of the fluid 46 entering the inlet pipe 10 in an easy and accurate manner. Thus, by means of the present invention, it is possible to provide a liquid measurement with sufficiently high accuracy.

The detection device adapted to collect small quantities of fluid 46 in a manner in which the volume of fluid 46 and the gas content can be measured with high accuracy.

Fig. 2 A) illustrates a detection device 2 corresponding to the one shown in Fig. 1. The detection device 2 is integrated in a tanker comprising a container 6 which is formed as an elongated tank. The detection device 2 has been brought into fluid communication with an external inlet tank 4. The external inlet tank 4 may be a farmer's milk tank.

In Fig. 2 A), the detection device 2 is ready to receive fluid 46 from the inlet tank 4 when the valves 12, 12" are opened. However, as the valves 12, 12" are closed, the fluid 46 will remain in the inlet tank 4 until the valve 12" is opened.

The valves 12', 16, 28 and 12'" are open, and the pipes 8, 8', 8", 10, 10', 10" are ready to receive the fluid 46 of the inlet tank 4. The pipe 8 is preferably formed as an inlet hose 8 for easy connection with the inlet tank 4.

When the inlet hose 8 has been attached to the inlet tank 4, the closed valve 12 ensures that no fluid 46 can enter the detection device 2 before desired.

When the fluid 46 is intended to be filled into the detection device 2, the valve 12" is opened as illustrated in Fig. 2 B).

In Fig. 2 B), it can be seen that the fluid 46 has entered the inlet hose 8, the gas separator 24 through the pipe 8' and the pipes 10, 10'.

In this stage, fluid 46 is filled into the central portion of the detection system 2. The valve 12' is closed, and the feeder pump 20 is activated in order to prime the main pump 18. Accordingly, fluid 46 flows into the gas separator 24 through the valve 12''' and further through the pipes 10, 10'.

It can be seen that the same level $L_1$ is achieved in the gas separator and in the pipe 10'.

The valves 16 and 28 are open, and the air in the pipes 10', 10'' is evacuated through the bottom in the container 6 via the pipe 10'''.

When a predefined fluid level (e.g. $L_1$) is achieved in the gas separator 24, the pump 18 will stop. This may be controlled by means of a control box (not shown).

Now, the detection device 2 is ready for the next stage as illustrated in Fig. 2 C).

In Fig. 2 C), the valve 12 is closed, whereas the valves 12', 12'', 12''', 16, 28 are open. In this process step, the fluid level $L_1$ is registered by the control unit (not shown). Now, the detection device 2 has knowledge about the volume of the fluid 46 in the pipes 8, 8', 10, 10', in the pumps 18, 20 and in the gas separator 24.

Subsequently, the detection device 2 is ready for the next stage as shown in Fig. 2 D). This stage corresponds to a scenario in which the detection device 2 is transported to the next farmer to collect milk 46 from a new inlet tank 4.

In Fig. 2 D), the flow sensors 32, 32' are reset, and the fluid level $L_1$ is registered before the valve 12 is opened and the valve 12''' is closed. Then, the valve 12'' is opened, and fluid 46 (e.g. milk) flows into the empty inlet hose 8.

The valve 12 is opened, while the valve 12''' is closed. The valve 28 is opened in a "floating position" in which position the valve 28 is open. The valve 28 creates a pressure drop corresponding to the pressure partly provided by a spring within the valve 28 and partly provided by an air pressure applied to the valve 28. The valve 12' is closed, and the feeder pump 20 is activated.

When a predefined quantity of fluid 46 (e.g. milk) has flowed through the first flow sensor 32, the detection device 2 is ready for the next stage as shown in Fig. 3 A).

In Fig. 3 A), the valve 28 is arranged in its "floating position", in which the valve 28 is open. The valve 28 causes a pressure drop corresponding to the pressure partly provided by the spring within the valve 28 provides and partly provided by an air pressure applied to the valve 28. The valve 12' is opened, and the main pump 18 is started.

The fluid level $L_2$ in the gas separator 24 is maintained within a predefined range (a first predefined set point and a second predefined set point) by means of the valve 16.

The detection device 2 is now in a "pumping in mode", and the detection device 2 will remain in this "pumping in mode" until there is no fluid 46 (e.g. milk) left to be collected from the inlet tank 4.

The fluid 46 flows into the gas separator 24 through the valve 12'. The feeder pump 20 prevents flow through the feeder pump 20, when the feeder pump 20 is inactive.

In this "pumping in mode", the gas/air content of the fluid 46 is given by the following equation and explained in the following section:

$$V_{air\,1} = \frac{P_2}{P_1} V_{air\,2}$$

The pressure $P_1$ of the fluid 46 entering the pipe 10 is detected by the pressure sensor 30. A first flow sensor 32 is arranged between the first pressure sensor 30 and the valve 28. The first flow sensor 32 is adapted to detect the flow $Q_1$ of the fluid 46 flowing through the pipe 10.

The valve 28 introduces a pressure drop which causes the second pressure sensor 30' arranged at the low pressure side of the valve 28 to detect a lower pressure $P_2$ than the pressure $P_1$ measured by means of the first pressure sensor 30. A second flow sensor 32' arranged next to the second pressure sensor 30' is adapted to detect the flow $Q_2$ of the fluid 46 flowing through the pipe 10'.

The flow sensors 30, 30' as well as the pressure sensors 32, 32' may be electrically connected to a control unit (not shown) by means of wires or by means of wireless connections. As the control unit 34 receives pressure and flow measurements from the flow sensors 30, 30' and the pressure sensors 32, 32', the control unit 34 can determine the gas/air percentage of the fluid.

As the liquid portion of the fluid 46 is incompressible, the flow $Q_{Liquid\,1}$ of the liquid entering the pipe 10 equals the flow $Q_{Liquid\,2}$ of the liquid leaving the pipe 10'. During a given time period, t, the volume of liquid $V_{Liquid\,1}$ flowing through the first pipe 10 equals the volume of liquid $V_{Liquid\,2}$ flowing through the second pipe 10'. This can be expressed in the following way:

$$(1) \qquad Q_{Liquid\ 1} = Q_{Liquid\ 2}$$

$$(2) \qquad tQ_{Liquid\ 1} = V_{Liquid\ 1} = V_{Liquid\ 2} = tQ_{Liquid\ 2}$$

As an approximation, one may assume that the air content of the fluid acts as an ideal gas. Accordingly, the ideal gas equation can be applied:

$$(3) \qquad PV = nRT,$$

P is the absolute pressure of the gas, V is the volume of the gas, n is the amount of substance of gas (measured in moles), T is the absolute temperature of the gas, and R is the ideal gas constant.

As T, R and n are constant during application of the device 2, it follows that the product between the pressure P and the volume V is constant. This can be expressed in the following way:

$$(4) \qquad PV = Constant$$

At the low pressure side of the pressure difference inducing member 40, it follows that:

$$(5) \qquad P_1 V_{air\ 1} = C, \text{ where } P_1 \text{ is the pressure, C is a constant, and } V_{air\ 1} \text{ is the volume of the air.}$$

At the high pressure side of the pressure difference inducing member 40, it follows that:

$$(6) \qquad P_2 V_{air\ 2} = C, \text{ where } P_2 \text{ is the pressure, C is the constant, and } V_{air\ 2} \text{ is the volume of the air.}$$

Combining (4) and (5), it follows that:

$$(7) \qquad P_1 V_{air\ 1} = P_2 V_{air\ 2} \text{ or expressed differently:}$$

$$(8) \qquad V_{air\ 1} = \frac{P_2}{P_1} V_{air\ 2}$$

Thus, the air flow $Q_{air\ 1}$ through the first flow sensor 32 is given by:

$$(10) \qquad Q_{air\ 1} = \frac{Q_2 - Q_1}{\frac{P_1}{P_2} - 1}$$

and

$$(11) \qquad V_{air\ 1} = \frac{V_2 - V_1}{\frac{P_1}{P_2} - 1}$$

Hence, the detection device 2 can be used to detect the air content of the fluid 46 entering the pipe 10 of the detection device 2.

The calculated amount of gas/air can be subtracted from the total volume/flow.

In one preferred embodiment according to the invention, the detection device 2 comprises a gas/air percentage and/or a liquid percentage.

The detection device 2 may comprise an alert unit configured to alarm the driver of a tanker when a gas/air percentage exceeding a predefined level is detected.

When the air content in the main pump 18 reaches a predefined level (causing the main pump to speed up), the main pump 18 is stopped. Now, the detection device 2 is ready for the next stage as shown in Fig. 3 B).

In Fig. 3 B), the valve 12 is opened, and the valve 28 is brought into its "floating position". At the same time, air pressure is applied to the valve 28. The feeder pump 20 is activated, and the valve 12' is closed. Subsequently, the inlet hose 8 and the inlet tank 4 are emptied so that no fluid 46 remains within the inlet hose 8 or the inlet tank 4.

Now, the valve 16 is controlled within a range defined by two predefined pressure levels (set points). In practice, the pressure levels are detected as (corresponding) fluid levels in the gas separator 24.

When the flow sensor 32 during this stage (process step) detects a flow below a predefined level (e.g. 20 L/min) in a time period exceeding a predefined level (e.g. 5 seconds), the valves 12 and 28 are closed, and the pump 18 is deactivated.

Now, the detection device 2 is ready for the next stage as shown in Fig. 3 C).

In Fig. 3 C), the valves 12, 28, 12" are closed, whereas the valves 12', 16 are open. The inlet hose 8 needs to be rolled up in order to empty the inlet hose 8 and finish the collection process.

In this stage (process step), it is possible to reactivate the pump 18 in order to empty the inlet hose 8 completely (like described with reference to Fig. 3 B).

Fig. 3 D) illustrates the final process step, in which the valves 12''', 12' are opened in order to "flood" the central portion of the detection device 2 with fluid 46 from the gas separator 24 in order to achieve well-defined and known start conditions when collecting fluid 46 (e.g. milk) from the next inlet tank (e.g. from the next farmer).

The fluid level $L_3$ in the gas separator is detected. The difference between the current fluid level $L_3$ and the start level $L_2$ can now be calculated. Based on this difference, $L_2 - L_3$, it is possible to adjust the collected fluid 46 measurement. Finally, the air content can be subtracted from the adjusted, collected fluid measurement in order to provide the most accurate measurement.

The next step corresponds to the one shown in Fig. 2 B). The valves 12, 28 are closed, whereas the valves 12', 12''', 16 are open. This process step can be considered as a "waiting condition" or "standby condition".

During the different process steps in which the pump is activated, it is possible to turn off the pump at any time desired and proceed to the stage shown in Fig. 3 C).

Due to the fact that the inlet hose 8 is emptied and that the fluid content in the detection device 2 is measured (fluid level $L_1$, $L_2$ and $L_3$), and that the gas separator 24 has a known geometry, it is possible to provide an accurate measurement of the collected fluid 46 even when small quantities of fluid 46 are collected.

Thus, by means of the invention, it is possible to provide a device and a method capable of providing accurate, fluid collection detection.

Moreover, the present invention makes it possible to provide a device and a method for collection of small quantities of fluid in a manner in which the volume can be measured with high accuracy.

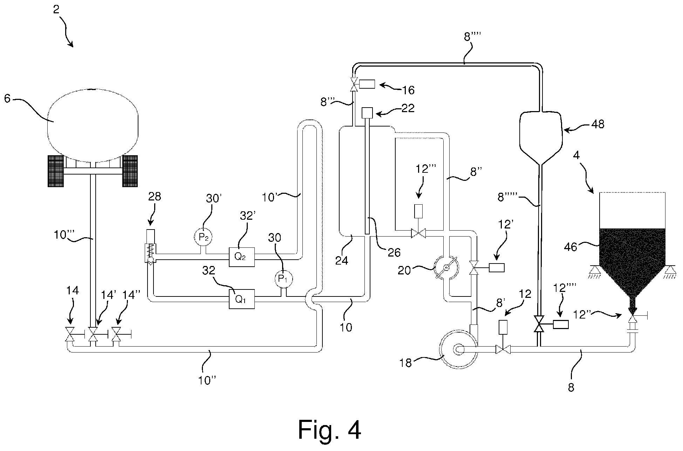

Fig. 4 illustrates a schematic view of a device 2 integrated within a tanker. The device 2 is almost identical to the one shown in Fig. 1.

An air evacuation pipe 8"" is connected to a vessel 48 which is in fluid communication with the pipe 8 by means of a pipe 8""'. The vessel 48 can collect foam from the gas separator 24. The evacuation pipe 8"" is connected to the gas separator 24 through a connection pipe 8"'. A valve 12"" is provided at the pipe 8""'.

The tanker comprises a container 6 configured to be brought into fluid communication with an (external) inlet tank 4. The inlet tank 4 may be e.g. a milk tank applied for milk storage at a farm.

The inlet tank 4 comprises a pipe 8 and a control valve 12" configured to be fully or partially opened or closed in order to adjust the fluid flow.

At the end of the pipe 8, a control valve 12 is provided. The pipe 8 is connected to a main pump 18 adapted to pump fluid from the inlet tank 4 into and through the detection device 2.

The detection device 2 comprises a gas separator 24 shaped as a tank provided with a dipstick 22 having a stick member 26. A pipe 8' connects the main pump 18 to a feeder pump 20 and a valve 12' controlling the degree of access to a bypass pipe (bypassing the feeder pump 18). An outlet pipe is provided at the bottom portion of the gas separator 24, and a valve 12''' is provided in order to close and open the outlet pipe (for the purpose of emptying the gas separator 24). A pipe 8" is connected to the upper portion of the gas separator 24.

An inlet pipe 10 connects the bottom portion of the gas separator 24 with a first pressure sensor 30 of the detection device 2. A first flow sensor 32 is provided next to the first pressure sensor 30. The first pressure sensor 30 detects a first pressure $P_1$, whereas the first flow sensor 32 detects a first flow $Q_1$.

A second pressure sensor 30' configured to detect a second pressure $P_2$ is arranged in fluid communication with the first flow sensor 32, and a check valve 28 is provided between the first flow sensor 32 and the second pressure sensor 30'. A second flow sensor 32' adapted to measure a second flow $Q_2$ is arranged next to the second pressure sensor 30'.

The second flow sensor 32' is provided at the outlet pipe 10' which is in fluid communication with a connection pipe 10" provided with three end valves 14, 14', 14" each configured to be connected to an inlet pipe 10''' which is connected to a container 6 of the tanker.

An air evacuation pipe 8"" is provided at the upper portion of the container 6 of the tanker. The evacuation pipe 8"" is connected to the gas separator 24 through a connection pipe 8'''. A valve 16 connects the evacuation pipe 8"" and the connection pipe 8'''. The detection device 2 may be shaped to evacuate air differently. The air may be evacuated to the atmosphere or into a vessel (e.g. a vessel for foam).

By using the detection device 2 in accordance with the invention, it is possible to detect the air content of the fluid entering the inlet pipe 10 in an easy and accurate manner. Thus, by means of the present invention, it is possible to provide a liquid measurement with sufficiently high accuracy.

The detection device is adapted to collect small quantities of fluid in a manner in which the gas content can be measured with high accuracy.

**List of reference numerals**

| | |
|---|---|
| 2 | Detection device |
| 4 | Inlet tank |
| 6 | Container of a tanker |
| 8, 8', 8", 8''', 8"", 8""' | Pipe |
| 10, 10', 10", 10''', 11 | Pipe |
| 12, 12', 12", 12''', 12"" | Valve |
| 14, 14', 14", 46 | Valve |
| 18 | Main pump |
| 20 | Feeder pump |
| 22 | Dipstick |
| 24 | Gas separator |
| 26 | Stick member |
| 28 | Valve |
| 30, 30' | Pressure sensor (manometer) |
| 32, 32' | Flow sensor |
| 34 | Control unit |
| 36 | Wire |
| 38 | Wireless signal |
| 40 | Pressure difference inducing member |
| 42, 42', 44, 44' | Arrow |
| 46 | Fluid |
| 48 | Vessel |
| $P_1$, $P_2$ | Pressure |
| t | Time |
| $\Delta P$ | Pressure difference |
| $Q_1$, $Q_2$, $Qair_1$, $Qair_2$ | Flow |
| $L_1$, $L_2$, $L_3$ | Level |
| $V$, $V_{Air\ 1}$, $V_{Air\ 2}$, $V_{Liquid\ 1}$, $V_{Liquid\ 2}$ | Volume |

**Claims**

1. Method for measuring the quantity of fluid (46) received by a detection device (2), which method comprises the following steps:

   - connecting the detection device (2) to an inlet tank (4);

- filling a quantity of fluid (46) from the inlet tank (4) into the detection device (2), wherein the quantity of fluid (46) exceeds a predefined volume;
- closing an outer valve (12) and flooding a portion of the detection device (2) hereby evacuating/removing the air in this portion of the detection device (2);
- detecting the fluid level ($L_1$) in the flooded portion of the detection device (2).

2. Method according to claim 1, **characterised in that** the method comprises the step of filling fluid (46) into a gas separator (24) and detecting the fluid level ($L_1$, $L_2$, $L_3$) in the gas separator (24).

3. Method according to claim 1 or claim 2, **characterised in that** the method comprises the step of:

   - opening the outer valve (12) and filling a further quantity of fluid (46) from the inlet tank (4) into the detection device (2), while detecting the flow and/or volume of the fluid (46);
   - emptying the inlet tank (4);
   - closing an outer valve (12) and flooding a portion of the detection device (2) hereby evacuating/removing the air in this portion of the detection device (2) and
   - detecting the fluid level ($L_2$, $L_2$) in the flooded portion of the detection device (2).

4. Method according to one of the preceding claims, **characterised in that** the method comprises the step of emptying an inlet hose (8) connected to the inlet tank (4).

5. Method according to one of the preceding claims, **characterised in that** the method comprises the step of applying one or more pumps (18, 20) to transport fluid (46) into and/or within the detection device (2).

6. Method according to claim 5, **characterised in that** the method comprises the step of emptying or filling the one or more pump(s) (18, 20).

7. Method according to one of the preceding claims, **characterised in that** the method comprises the step of determining the pressure at both sides of a pressure difference inducing member (40, 28) provided in the detection device (2), wherein the method further comprises the step of determining the flow ($Q_1$, $Q_2$) at each side of the pressure difference inducing member (40, 28), and applying the pressures and flows at both sides of the pressure difference inducing member (40, 28) to calculate the air/gas content of the fluid (46).

8. Method according to one of the preceding claims, **characterised in that** the method comprises the step of flooding a portion of the detection device (2) when the inlet tank (4) has been emptied, and the remaining quantity of fluid (46) in the detection device (2) has been detected.

9. Use of the method according to any one of claims 1-8 in a tanker.

10. Use according to claim 9, **characterised in that** the tanker is a milk tanker.

11. A tanker comprising a detection device (2) for measuring the quantity of fluid (46) received by the detection device (2), wherein the detection device (2) is configured to carry out the method according to one of the claims 1-8, wherein the detection device (2) is configured to be connected to an inlet tank (4), wherein the detection device (2) is configured to receive a quantity of fluid (46) from the inlet tank (4) being filled into the detection device (2), wherein the quantity of fluid (46) exceeds a predefined volume, wherein the detection device (2) comprises an outer valve (12) configured to be brought from an open configuration into a closed configuration, wherein the detection device (2) is configured to be flooded in at least a portion of the detection device (2) in order to evacuate/remove the air in this portion of the detection device (2), wherein the detection device (2) comprises means for detecting the fluid level ($L_1$) in the flooded portion of the detection device (2).

12. A tanker according to claim 11, **characterised in that** the detection device (2) comprises a gas separator (24) and means for detecting the fluid level ($L_1$, $L_2$, $L_3$) in the gas separator (24), wherein the gas separator (24) is configured to receive fluid (46) being filled into the gas separator (24).

13. A tanker according to claim 11 or claim 12, **characterised in that** the tanker comprises means for detecting the flow and/or volume of a fluid (46) filled from the inlet tank (4) into the detection device (2).

**14.** A tanker according to claim 11 or claim 13, **characterised in that** the tanker comprises one or more pumps (18, 20) arranged and configured to transport fluid (46) into and/or within the detection device (2).

**15.** A detection device (2) which may preferably be used in a tanker for measuring the quantity of fluid (46) received by the detection device (2), which device (2) comprises:

- a gas separator (24) and a level detection member configured to detect the fluid level ($L_1$, $L_2$, $L_3$) of the gas separator (24);
- a connection member (8) for connecting the detection device (2) to an inlet tank (4);
- a filling member (18, 20) for filling a quantity (within a predefined range) of fluid (46) from the inlet tank (4) into the detection device (2), the quantity of fluid (46) exceeding a predefined volume;
- an outer valve (12) arranged between the filling member (18, 20) and the distal end of the connection member (8);
- a first flow sensor (32) and a second flow sensor (32') arranged below the gas separator (24).
- a main pump (18) adapted to pump fluid (46) from the inlet tank (4) into and through the detection device (2);
- a feeder pump (20) connected to the main pump (18) via a pipe (8');
- a valve (12') arranged between the main pump (18) and the gas separator (24), which valve (12') is adapted to control the degree of access to a bypass pipe bypassing the feeder pump (20) via the pipe (8');
- a valve (12''') provided in order to close and open an outlet pipe of the gas separator (24) for the purpose of flooding the pump (18, 20).

Fig. 1

Fig. 2

Fig. 3

Fig. 4

Europäisches
Patentamt

European
Patent Office

Office européen
des brevets

## EUROPEAN SEARCH REPORT

Application Number

EP 16 15 1852

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/132451 A1 (BOEHM ALFRED [DE] ET AL) 3 June 2010 (2010-06-03) | 1-6,8-15 | INV. A01J5/01 |
| Y | * paragraphs [0012], [0019], [0063], [0070] - [0091]; figures 2,4 * | 7 | B67D7/08 G01F1/74 G01F15/08 |
| Y | DE 10 2005 005295 A1 (BARTEC GMBH [DE]) 22 June 2006 (2006-06-22) | 7 | G01F23/00 |
| A | * paragraph [0060]; figure 5 * | 1,15 | |

----- 

TECHNICAL FIELDS
SEARCHED (IPC)

A01J
B67D
G01F
G01N

The present search report has been drawn up for all claims

| Place of search | Date of completion of the search | Examiner |
|---|---|---|
| The Hague | 14 June 2016 | Myrillas, K |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.......................................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 16 15 1852

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2016

| Patent document cited in search report | Publication date | Patent family member(s) | Publication date |
|---|---|---|---|
| US 2010132451 A1 | 03-06-2010 | AT 516702 T<br>CA 2683755 A1<br>EP 2148563 A1<br>KR 20100017231 A<br>PL 2148563 T3<br>US 2010132451 A1<br>WO 2008141664 A1 | 15-08-2011<br>27-11-2008<br>03-02-2010<br>16-02-2010<br>31-01-2012<br>03-06-2010<br>27-11-2008 |
| DE 102005005295 A1 | 22-06-2006 | NONE | |

EPO FORM P0459

For more details about this annex : see Official Journal of the European Patent Office, No. 12/82

**EP 3 050 430 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8304407 A1
- GB 2432680 A
- GB 2240764 A
- EP 80851 A1